# FASCICULE DE BREVET EUROPEEN

(11) **EP 1 476 103 B1**
(45) Date de publication et mention de la délivrance du brevet: **30.09.2009**
(21) Numéro de dépôt: 03702226.6
(22) Date de dépôt: 12.02.2003
(51) Int. Cl.: A61F 6/14

(54) **DISPOSITIF INTRA-UTERIN EN T ET SON PROCEDE DE FABRICATION**
T-FÖRMIGE INTRAUTERINE VORRICHTUNG UND DEREN HERSTELLUNGSVERFAHREN
T-SHAPED INTRA-UTERINE DEVICE AND METHOD FOR THE PRODUCTION THEREOF

(30) Priorité: 12.02.2002 BE 200200088
(43) Date de publication de la demande: 17.11.2004
(73) Titulaire: Wildemeersch, Dirk, 8301 Knokke-Heist (BE)
(72) Inventeur: Wildemeersch, Dirk, 8301 Knokke-Heist (BE)
(74) Mandataire: Colens, Alain M.G.M.
(86) Numéro de dépôt international: PCT/BE2003/000023
(87) Numéro de publication internationale: WO 2003/068117

(56) Documents cités:
- EP-A- 0 147 274
- WO-A-00/67684
- WO-A-96/01092
- US-A- 3 971 367
- US-A- 4 093 708
- US-A- 5 224 493
- US-A- 5 433 218

## Description

La présente invention a pour objet un dispositif intra-utérin du type en T. Elle a également pour objet le procédé de fabrication d'un tel dispositif intra-utérin.

Plus particulièrement l'invention a pour objet un dispositif intra-utérin de conception nouvelle et de réalisation particulièrement peu coûteuse, adoptant la forme conventionnelle d'un T et permettant la libération d'au moins une substance active, tout en étant, à dimensions égales, susceptible de renfermer une quantité plus importante de substance active que les stérilets en T connus, et donc de présenter, en termes de libération de substance active, une plus longue durée d'activité.

Les dispositifs intra-utérins en T sont connus de longue date. Ils sont réalisés d'une seule pièce, en matière moulée, et portent généralement, sur au moins un des bras, un élément rapporté libérant une substance active.

Dans le domaine des dispositifs intra-utérins libérant des substances actives telles des stéroïdes, il est également connu de réaliser le dispositif libérant les stéroïdes sous la forme d'une fibre constituée d'une membrane poreuse entourant un noyau contenant la substance active.

Il a déjà été proposé de réaliser, à l'aide de telles fibres, des dispositifs intra-utérins sous la forme d'une fibre solidaire d'un fil pourvu d'un moyen de fixation à la paroi de la matrice. De tels dispositifs, s'ils se sont révélés efficaces, sont cependant relativement délicats à mettre en oeuvre par un personnel peu qualifié, en raison de leur fixation à la paroi de la matrice.

Quant à l'usage de stéroïdes dans des dispositifs conventionnels, notamment des dispositifs intra-utérins en T du type précité, il a déjà été proposé soit d'enrouler, comme cela se fait avec des filaments métalliques, une fibre souple libérant des stéroïdes (demande PCT WO 00/67684), sur au moins un des bras du "T", soit encore de former dans le "T" des cavités destinées à recevoir la matière active (EP 0117163), soit encore de sertir sur un des bras du "T" une capsule contenant la matière active. Ces diverses réalisations, outre le fait qu'elles entraînent une fabrication relativement compliquée et coûteuse, aboutissent à des dispositifs encombrants, dont l'introduction dans la matrice, notamment de jeunes femmes n'ayant pas encore enfanté, ne se fait pas aisément. Ils sont également limités quant à la quantité de substance active qu'ils sont susceptibles de recevoir, et donc quant à leur durée d'activité.

WO-A-96-1092 montre un dispositif contraceptif intra-uterin dans la forme d'un T.

La présente invention a pour but de remédier à ces divers inconvénients en prévoyant un dispositif intra-utérin de faible encombrement, susceptible de contenir une grande quantité de matière active, de réalisation peu coûteuse, permettant l'usage d'un dispositif d'insertion de section extrêmement réduite, et demandant peu de manipulation à l'insertion.

Ce but est atteint en prévoyant un dispositif intra-utérin, en T comportant une branche longitudinale constituant le corps du T; à la partie supérieure de laquelle se raccorde une branche transversale constituant les bras du T, un fil d'extraction étant fixé à la partie inférieure du corps du T, dans lequel au moins le corps du T est constitué d'une fibre libérant une substance active, et les bras sont rapportés sur ce corps,
- la fibre constituant le corps du T présente à sa partie supérieure un canal transversal, et les bras sont constitués d'une tige, de section inférieure à celle de la fibre mais supérieure à celle du canal, insérée à force dans le dit canal pour former de part et d'autre du corps deux bras égaux et dans lequel
- la tige formant les bras présente une forme incurvée, et l'effort de serrage exercé par le corps sur la tige est tel que cette dernière est normalement retenue dans le canal dans la position où elle y a été introduite, c'est-à-dire une position où les bras sont orientés vers le bas du corps, tandis qu'elle est autorisée à pivoter dans le canal jusqu'à ce que les bras soient orientés vers le haut du corps sous l'effet des contraintes exercées sur les bras lors de l'extraction du dispositif de la matrice.

Selon d'autres modes particuliers de réalisation du dispositif de l'invention
- le corps est constitué par l'assemblage bout à bout de divers tronçons de fibres, libérant chacun une matière active différente.
- la tige constituant les bras est également constituée d'une fibre libérant une substance active.

Un procédé de réalisation d'un dispositif intra-utérin en T suivant l'invention consiste à réaliser une fibre libérant des stéroïdes de section et de caractéristiques de libération correspondant à la section et aux caractéristiques de libération souhaitées pour le corps du dispositif intra-utérin, à tronçonner cette fibre en sections de longueur correspondant à la longueur souhaitée pour la partie du corps du dispositif intra-utérin qu'elle constitue, à former à la partie supérieure d'une section constituant le corps du dispositif intra-utérin un canal destiné à recevoir là tige constituant les bras, et à rendre le corps solidaire de la tige au droit de ce canal

Selon d'autres caractéristiques du procédé:
- on forme le canal destiné à recevoir les bras dans la partie supérieure du corps par perçage, et on introduit la tige constituant les bras à force dans le dit canal
- pour la réalisation d'un dispositif intra-utérin libérant plusieurs substances actives, on découpe dans diverses fibres, chacun libérant une des substances actives suivant les caractéristiques souhaitée, des sections de fibres que l'on assemble ensuite bout à bout, par collage, dans l'ordre souhaité, afin de constituer le corps du dispositif intra-utérin.

Enfin, un kit pour la pose d'un dispositif intra-utérin selon l'invention est **caractérisé en ce qu**'il est constitué, sous conditionnement stérile, d'un inserteur constitué d'un tube, d'un diamètre interne égal ou légèrement supérieur au diamètre externe du dit corps du T, dans lequel est logé le corps du dispositif intra-utérin en T.

L'invention sera mieux comprise en se reportant à la description en même temps qu'au dessin annexé qui représente, uniquement à titre d'exemple, divers modes de réalisation de l'invention et dans lequel :
- la fig.1 représente, partiellement en coupe, un mode de réalisation de dérivé de l'art antérieur
- les fig 2a et 2b sont des vues de détail, agrandies, respectivement d'un mode de fixation préféré des bras au corps du dispositif, à la partie supérieure de celui-ci, et d'un mode de fixation préféré du fil d'extraction au corps à la partie inférieure de celui-ci

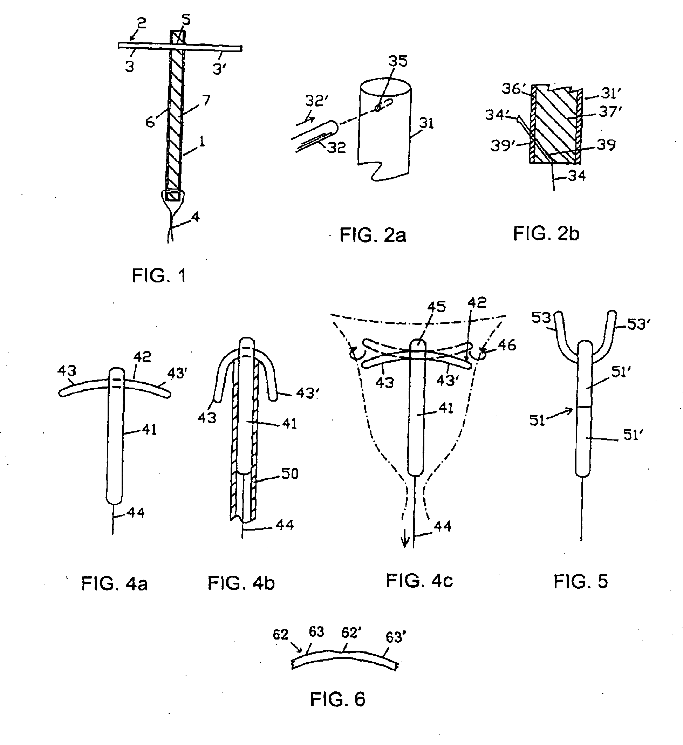

La figure 2 originale a eté effacée.
- les fig 4a, 4b, 4c sont diverses vues d'un mode de réalisation de l'invention dans différentes positions des bras
- la fig 5 est une vue d'un mode de réalisation de l'invention similaire à celui illustré aux fig 4a à 4c, adapté à la libération de deux substances actives différentes dans la matrice, le dispositif étant illustré dans encore une autre position des bras
- la fig 6 est une vue de détail d'un autre mode de réalisation de la tige constituant les bras.

En se reportant à la fig.1, le dispositif intra-utérin est constitué d'une branche longitudinale constituant le corps 1 du dispositif, d'une branche transversale 2 en forme de tige constituant les bras 3, 3' du dispositif, et d'un fil d'extraction 4.

Le corps 1 est constitué d'une fibre libérant une substance active dans la matrice. Par « fibre » on entend dans la présente description un élément réalisé suivant la technique des fibres connues dans le domaine de la contraception, libérant par exemple des progestagènes. De telles fibres sont constituées d'une enveloppe perméable en forme de membrane 6, et d'un noyau 7 contenant, dans un support approprié, la substance active destinée à être diffusée au travers de la membrane. La fibre utilisée dans le dispositif de la présente invention se distingue toutefois des fibres connues par l'importance de sa section transversale qui correspond à la section transversale souhaitée pour le corps 1 du T.

Ainsi, c'est l'ensemble du corps 1 qui constitue à la fois réservoir de la matière active, et surface de diffusion de la matière active. Ces deux aspects assurent au dispositif ainsi constitué, d'une part une grande capacité de stockage de matière active, et donc une longue durée d'activité, et d'autre part une surface de diffusion de la matière active répartie sur toute la surface externe du corps, ce qui assure une bonne répartition de la matière active dans la matrice.

Sur ce corps 1 sont rapportés des bras 3, 3'. Dans le mode de réalisation illustré en fig. 1 ceci est réalisé par introduction de la tige 2 dans un canal 5 formé à la partie supérieure du corps 1.

Des détails de modes de réalisation préférés sont illustrés aux figures 2a et 2b.

Plus particulièrement à la fig. 2a est illustré, en perspective, un mode de réalisation préféré du procédé de montage de la tige 32, destinée à former les bras, sur le corps 31. Suivant ce mode de réalisation on perce à la partie supérieure du corps 31 un canal ou logement 35 dont le diamètre est inférieur au diamètre de la tige 32, celui-ci étant lui-même inférieur au diamètre du corps 31. La tige 32 est ensuite introduite à force dans le canal 35 suivant la flèche 32', jusqu'à ce que la tige 32 s'étende sensiblement symétriquement de part et d'autre du corps 31 pour former les bras. La tige 32 est ainsi maintenue sur le corps 31 par serrage dans le canal 35.

La symétrie des bras est souhaitable, pour correspondre à la symétrie de la matrice en assurant un bon positionnement du corps actif dans la matrice, sensiblement dans l'axe de celle-ci. Il faut cependant remarquer que la fonction des bras est de maintenir le corps libérant la substance active sensiblement dans l'axe de la matrice, avec une extrémité proche du fond de la matrice et l'extrémité opposée proche du col, et que cette fonction sera remplie, même si les bras ne sont qu'approximativement d'égale longueur.

La fig 2b illustre, en coupe, le détail d'un mode de montage du fil d'extraction 34 sur le corps 31. Suivant ce mode de réalisation on forme à la partie inférieure du corps 31', au travers de la membrane 36' et du noyau 37' un canal 39, dont la partie 39' formée dans la membrane 36' est élargie. On élargit, par exemple par déformation à chaud, l'extrémité 34' du fil 34 et on insère le fil 34 dans le canal 39 jusqu'à ce que son extrémité élargie 34' vienne se bloquer dans la partie élargie 39' du canal 39. Ce mode de réalisation permet de noyer totalement le dispositif de retenue du fil d'extraction 34' dans le corps 31', et ainsi de limiter strictement l'encombrement de ce dernier aux dimensions de sa section transversale.

Les fig. 4a, 4b et 4c sont des vues, dans diverses positions, du même mode de réalisation préféré de l'invention.

La fig. 4a illustre le dispositif intra-utérin dans sa position normale d'utilisation. Ce dispositif est constitué d'un corps 41 à la partie supérieure duquel a été insérée, dans un canal tel qu'illustré à la fig 3a, une tige 42 incurvée formant des bras 43, 43', cette tige incurvée étant retenue dans le canal dans une position où les bras 43, 43' sont dirigés vers le bas du corps 41. Un fil d'extraction 44 est retenu à la partie inférieure du corps 41 de la manière illustrée à la fig 2b.

La fig. 4b montre le dispositif intra-utérin, avec son inserteur 50, qui se présente sous forme d'un tube dans lequel le corps 41 est enfoncé jusqu'au niveau des bras 43, 43' et dans lequel passe également le fil d'extraction 44. Dans cette figure les bras 43, 43' sont représentés repliés le long de l'inserteur 50, dans la position qu'ils adoptent lors du passage du col de la matrice, sous l'effet de l'effort d'introduction. Il y a lieu de remarquer que, suivant ce mode de réalisation préféré de la présente invention, seul le corps du T est repris dans l'inserteur, tandis que les bras demeurent à l'extérieur de l'inserteur et se courbent le long de celui-ci lors de leur passage du col de l'utérus. Ceci permet une réduction importante de la section de l'inserteur utilisé, par comparaison avec la plupart des dispositifs d'insertion de dispositifs intra-utérins conventionnels en T, dans lesquels les bras du T, en raison de leur rigidité, sont repliés dans l'inserteur, ce qui aboutit à des dispositifs d'insertion de section importante. Dans ces dispositifs conventionnels connus, le fait que le T soit intégralement contenu dans l'inserteur exige aussi l'usage d'un poussoir pour expulser le stérilet du tube inserteur. Par contre, suivant le mode de réalisation illustré de la présente invention, la faible section de la tige 42 constituant les bras 43, 43' confère à ceux-ci une élasticité suffisante pour qu'ils se plient le long de l'inserteur au passage du col de l'utérus, et reprennent ensuite dans la matrice leur position normale. Une fois que les bras 43, 43' ont repris leur position déployée dans la matrice, ils assurent d'eux-mêmes, par leur contact avec les parois de la matrice, une force de retenue permettant le glissement du corps 41 hors de l'inserteur 50 lors du retrait de ce dernier. La réduction du nombre d'opérations lors de l'insertion assure d'une part une réduction des risques de contamination bactérienne, et d'autre part une simplification de la procédure d'insertion, tandis que la réduction de la période au cours de laquelle les bras sont pliés (limitée au temps de passage du col de l'utérus) favorise un retour quasi instantané des bras à leur position initiale, et évite tout risque de déformation permanente susceptible de favoriser une expulsion de dispositif intra-utérin.

Enfin la fig. 4c montre le même dispositif intra-utérin en place dans la matrice représentée en traits d'axe. A l'issue de la procédure d'introduction, les bras 43, 43' reprennent la position représentée en traits pleines, qu'ils conservent tout au long de la période d'utilisation du dispositif intra-utérin, même lorsqu'ils sont soumis aux contraintes résultant des mouvements de la matrice. Par contre, sous l'effet d'un effort plus important, tel celui que les parois de la matrice exercent à l'extrémité des bras 43, 43' lorsqu'une traction est exercé sur le fil 44, les bras pivotent dans leur canal 45 comme indiqué par les flèches 46, jusqu'à adopter une position à 180° de la première, c'est-à-dire avec les bras s'incurvant vers le haut, ce qui facilite l'extraction du dispositif hors de la matrice. A titre d'exemple, le serrage des bras sera conçu pour permettre leur pivotement dans la matrice à partir d'une traction de l'ordre 5 Newtons exercée sur le fil de traction.

La fig.5 montre un autre mode de réalisation d'un dispositif de l'invention, conçu pour délivrer dans la matrice deux matières actives différentes. Dans ce but le corps 51 est réalisé à partir de deux segments de fibres différents 51', 51", chacun obtenu à partir d'une fibre contenant la matière active souhaitée. Les segments 51', 51" sont assemblés bout à bout par collage. Les bras 53, 53' sont représentés dans les positions qu'ils occupent lors de l'extraction du dispositif de la matrice, après pivotement comme illustré à la fig 4c, et étant rabattus par l'effort d'extraction lors du passage du col de la matrice.

La fig 6 enfin montre un mode de réalisation possible d'une tige 62 destinée à former les bras 63, 63' d'un dispositif intra-utérin suivant l'invention. Suivant ce mode de réalisation la tige 62 présente en son centre une déformation 62' destinée à coopérer avec le canal (non représenté) formé dans le corps du dispositif. Le diamètre de la tige 62 à l'endroit de la déformation sera de préférence égal ou légèrement inférieur au diamètre du canal, et la longueur de la déformation correspondra sensiblement à la longueur du canal, de manière à faciliter le pivotement des bras, tout en empêchant tout déplacement latéral sensible des bras par rapport au canal.

La fibre ou le tronçon de fibre constituant le corps (1; 21; 31; 41) peut être ouvert à ses extrémités supérieure et inférieure, en ce sens que le noyau (7) contenant la substance active peut y être nu, ou exposé. Le taux de libération de matière active par le noyau 7 nu est plus élevé que le taux de libération à travers la membrane poreuse 6, puisque celle-ci rempli une fonction de contrôle à cet égard. Ceci peut être avantageux lorsque l'on souhaite par exemple délivrer plus de substance active dans la région du fond et dans la région du col de la matrice.

La fibre ou tronçon de fibre constituant le corps (1; 21; 31; 41) peut également être fermé à ses extrémités supérieure et inférieure, ce qui peut être obtenu, par exemple par pinçage des extrémités tronçonnées, éventuellement avec apport de chaleur pour assurer une jonction ou une soudure ou moins partielle des lèvres de la membrane 6, à ces extrémités, de façon à produire une fibre assurant une diffusion sensiblement uniforme sur toute son étendue.

En ce qui concerne les dimensions du dispositif intra-utérin de l'invention, celles-ci se situeront avantageusement dans les intervalles suivants:
- longueur de la tige (2; 22; 32; 42) constituant les bras: 24 - 32 mm
- diamètre de la tige (2; 22; 32; 42) constituant les bras: 1 - 2 mm
- longueur du corps (1; 21; 31; 41; 51): 20 - 40 mm
- diamètre du corps (1; 21; 31; 41; 51): 1,8 - 3,0 mm
- force nécessaire pour faire pivoter les bras: 1 - 10 Newton

Selon un mode de réalisation préféré, ces dimensions sont les suivantes:
- longueur de la tige (2; 22; 32; 42) constituant les bras: ± 28 mm
- diamètre la tige (2; 22; 32; 42) constituant les bras: ± 1,5 mm
- longueur du corps (1; 21; 31; 41; 51): ± 30 mm
- diamètre du corps (1; 21; 31; 41; 51): ± 2,4 mm
- force nécessaire pour faire pivoter les bras: ± 5 Newton

Un dispositif présentant des dimensions selon le mode de réalisation préféré, utilisé en tant que dispositif anticonceptionnel, et contenant du levenorgestrel comme matière active aura une durée d'activité active calculée de l'ordre de 10 ans, pour un taux de libération du levenorgestrel de 14-20 µg/jour.

A titre de comparaison, les dispositifs intra-utérins en T du type à fibre enroulée ou à capsule, comme discuté dans l'introduction, présentent dans les mêmes conditions une durée d'activité active calculée allant d'environ 1 à maximum 5 ans.

Comme on peut le constater à la lecture de la description ci-dessus, et du dessin annexé, on a réalisé selon l'invention un dispositif intra-utérin en T, combinant les avantages associés à ce type de dispositif (par exemple facilité de pose, manipulation réduite, et bon positionnement dans la matrice), et ceux d'une fibre connue (quantité de matière active, et durée d'activité), le tout sous des dimensions extrêmement réduites.

Selon l'invention, ceci est obtenu en rapportant sous la forme de bras, une tige qui peut être très fine (1 mm), et sera généralement en matière synthétique, sur une fibre de structure en soi connue, formant le corps d'un stérilet en T.

Dans le dispositif intra-utérin de l'invention, la fibre réservoir de matière active présente un section supérieure à celle des fibres utilisées jusqu'à présent, dans le but de lui assurer une plus grande capacité de stockage de matière active, et également de lui conférer une plus grande rigidité.

Bien que cela n'ait pas été décrit dans ce qui précède, il va de soi que la tige constituant les bras peut également être constituée d'une fibre de la même structure que le corps, imprégnée d'une substance active qui peut être ou non différente de celle contenue dans le corps. Ceci permet d'accroître encore la capacité d'emmagasinage du dispositif en substance active, et également de renforcer le taux de diffusion de substance dans la partie supérieure de la matrice. Lorsque les extrémités de la fibre formant les bras sont "ouvertes" au sens mentionné plus haut (noyau imprégné nu, ou exposé), le taux de diffusion sera en particulier renforcé en direction des trompes, ce qui peut être favorable.

## Revendications

1. Dispositif intra-utérin en T comportant une branche longitudinale (41) constituant le corps du T, à la partie supérieure de laquelle se raccorde une branche transversale (42) constituant les bras (43, 43') du T, un fil d'extraction (44) étant fixé à la partie inférieure du corps du T, dans lequel
au moins le corps (41) du T est constitué d'une fibre libérant une substance active, et en ce que les bras (43, 43') sont rapportés sur ce corps,
la fibre constituant le corps du T présente à sa partie supérieure un canal transversal (45), et les bras sont constitués d'une tige, de section inférieure à celle de la fibre mais supérieure à celle du canal, apte à être insérée à force dans le dit canal (45) pour former de part et d'autre du corps (41) deux bras égaux (43, 43'), et dans lequel
la tige (42) formant les bras présente une forme incurvée, et en ce que l'effort de serrage exercé par le corps sur la tige est tel que cette dernière est normalement retenue dans le canal dans la position où elle y a été introduite, c'est-à-dire une position où les bras sont orientés vers le bas du corps, tandis qu'elle est autorisée à pivoter dans le canal jusqu'à ce que les bras soient orientés vers le haut du corps sous l'effet des contraintes exercées sur les bras lors de l'extraction du dispositif de la matrice.

2. Dispositif suivant la revendication 1 **caractérisé en ce que** le corps (51) est constitué par l'assemblage bout à bout de divers tronçons de fibres (51', 51"), libérant chacun une matière active différente.

3. Dispositif suivant l'une quelconque des revendications 1 à 2, **caractérisé en ce que** la tige (42) constituant les bras (43, 43') est également constituée d'une fibre libérant une substance active.

4. Procédé de réalisation d'un dispositif intra-utérin en T suivant l'une des revendications précédentes, **caractérisé en ce qu'**il consiste à réaliser une fibre libérant des stéroïdes de section et de caractéristiques de libération correspondant à la section et aux caractéristiques de libération souhaitées pour le corps du dispositif intra-utérin, à tronçonner cette fibre en sections de longueur correspondant à la longueur souhaitée pour la partie du corps du dispositif intra-utérin qu'elle constitue, à former à la partie supérieure d'une section constituant le corps (1) du dispositif intra-utérin un canal (5) destiné à recevoir la tige constituant les bras, et à rendre le corps solidaire de la tige au droit de ce canal.

5. Procédé suivant la revendication 4, **caractérisé en ce que** l'on forme le canal destiné à recevoir les bras dans la partie supérieure du corps par perçage, et **en ce que** l'on introduit la tige (2) constituant les bras à force dans le dit canal.

6. Procédé suivant la revendication 4, **caractérisé en ce que**, pour la réalisation d'un dispositif intra-utérin libérant plusieurs substances actives, on découpe dans diverses fibres, chacun libérant une des substances actives suivant les caractéristiques souhaitées, des sections de fibres (51', 51") que l'on assemble ensuite bout à bout, par collage, dans l'ordre souhaité, afin de constituer le corps (51) du dispositif intra-utérin.

7. Kit pour la pose d'un dispositif intra-utérin selon l'une quelconque des revendications 1 à 3, **caractérisé en ce qu'**il est constitué, sous conditionnement stérile, d'un inserteur (50) constitué d'un tube, d'un diamètre interne égal ou légèrement supérieur au diamètre externe du dit corps du T, dans lequel est logé le corps du dispositif intra-utérin en T.

## Claims

1. T-shaped intrauterine device including a longitudinal branch (41) constituting the body of the T, to an upper part of which is connected a transversal branch (42) constituting the arms (43, 43') of the T, an extraction wire (44) being fixed to a lower part of the body of the T, wherein
at least the body (41) of the T is made up of a fibre that releases an active substance and, the arms (43, 43') are connected to the body,
the fibre constituting the body of the T presents a transverse channel (45) on its upper part, and the arms are made up of a stem, with a section inferior to that of the fibre but superior to that of the channel (45), inserted by force into said channel to form two equal arms on opposite side of the body (43, 43') and wherein
the stem (42) forming the arm presents a curved form and in that the wedging force exercised by the body on the stem is such that the latter is usually held in the channel in the position where it has been introduced, that is to say a position where the arms are oriented downwards of the body, while is it allowed to pivot in the channel until the arms are oriented upwards of the body under the effect of constraint exercised on the arms at the time of the extraction of the device from the womb.

2. Device according to claim 1 **characterized in that** the body (51) is constituted by the end to end assembly of different fibre sections (51, 51'), each releasing a different active substance.

3. Device according to any of claims 1 to 2, **characterized in that** the stem (42) constituting the arms (43, 43') is also made up of a fibre that releases an active substance.

4. Manufacturing process of a T-shaped intrauterine device according to one of the previous claims, **characterized in that** it consists
in producing a fibre that releases section-specific steroids with release characteristics relating to the section and with the desired release characteristics for the body of the intrauterine device,
in cutting-up this fibre into sections of length corresponding to the length desired for the part of the body of the intrauterine device that it constitutes,
in forming a channel (5) on the upper part of a section constituting the body (1) of the intrauterine device for receiving the stem constituting the arms and in attaching the body to the stem perpendicularly in this channel.

5. Process according to claim 4, **characterized in that** a channel is formed that is intended to receive the arms in the upper part of the body by drilling, and **in that** the stem (2) constituting the arms is introduced by force into said channel.

6. Process according to claim 4, **characterized in that** the channel intended to receive the arms is formed on the upper part of the body by grooving, and **in that** the stem constituting the arm is introduced there and is maintained there by gluing.

7. Kit for the positioning of an intrauterine device according to any of the claims 1 to 3, **characterized in that** it is constituted, under sterile packaging, of an inserter (50) made up of a tube, with an internal diameter equal to or slightly higher than the external diameter of said T-shaped body, in which the T-shaped body of the intrauterine device is housed.

## Patentansprüche

1. Intra-uterine Vorrichtung in T-Form, die einen den Korper des T bildenden Längsschenkels (41) umfasst, an dessen oberen Teil ein Querschenkel (42), der die Arme (43, 43') des T bildet, angeschlossen ist, wobei ein Ausziehfaden (44) an dem oberen Teil des Korpus des T befestigt ist,
bei der
mindestens der Körper (41) des T aus einer Faser besteht, die eine wirksame Substanz freigibt, und die Arme (43, 43') auf diesem Körper angesetzt sind,
die Faser, die den Körper des T bildet, an ihrem oberen Teil einen Querkanal (45) aufweist, und die Arme aus einer Stange besteht, deren unterer Querschnitt kleiner ist als derjenige der Faser, jedoch größer als derjenige des Kanals und so ausgebildet ist, dass sie in den besagten Kanal (45) zwangsschlüssig eingeführt werden kann, um beidseitig des Körpers (41) zwei gleiche Arme (43, 43') zu bilden
und bei der
die Stange (42), die die Arme bildet, eine einwärts gekrümmte Form aufweist und die Spannkraft, die durch den Körper auf die Stange ausgeübt wird, derart ist, dass die Letztgenannte im Normalfall in dem Kanal, in der Position, in der sie darin eingeführt wurde, das heißt in einer Position, in der die Arme zu dem unteren Teil des Körpers hin gerichtet sind, zurückgehalten wird, während sie die Möglichkeit hat, sich in dem Kanal zu drehen, bis die Arme unter der Wirkung der Kräfte, denen sie bei dem Herausziehen der Vorrichtung aus der Gebärmutter ausgesetzt sind, zum oberen Teil des Körpers hin gerichtet sind.

2. Vorrichtung nach dem Anspruch 1, **dadurch gekennzeichnet, dass** der Körper (51) aus auf Stoß miteinander verbundenen verschiedenen Faserstücken (51', 51") zusammengesetzt ist, die jeweils eine unterschiedliche wirksame Substanz freigeben.

3. Vorrichtung nach irgendeiner der Ansprüche 1 und 2, **dadurch gekennzeichnet, dass** die Stange (42), die die Arme (43, 43') bildet, ebenfalls aus einer Faser besteht, die eine wirksame Substanz freigibt.

4. Verfahren zur Realisierung einer intra-uterinen Vorrichtung in T-Form nach einem der vorangegangenen Ansprüche, **dadurch gekennzeichnet, dass** es darin besteht,
- eine Faser herzustellen, die Steroide freisetzt, deren Querschnitt und Freigabemerkmale dem Querschnitt und den Freigabemerkmalen entsprechen, die für den Körper der intra-uterinen Vorrichtung gewünscht sind,
- diese Faser in Stücke zu zerlegen, deren Länge der Länge entspricht, die für den Teil des Körpers der intra-uterinen Vorrichtung, den sie bildet, erwünscht ist, am oberen Teil eines Stückes, das den Körper (1) der intra-uterinen Vorrichtung bildet, einen Kanal (5) zu formen, der dazu bestimmt ist, die Stange, die die Arme bildet, aufzunehmen,
- und den Körper mit der Stange lotrecht zu diesem Kanal einstückig zu verbinden.

5. Verfahren nach dem Anspruch 4, **dadurch gekennzeichnet, dass** man den Kanal, der dazu bestimmt ist, die Arme aufzunehmen, in dem oberen Teil des Körpers durch Bohrung formt, und dass man unter Kraftanwendung die Stange (2), die die Arme bildet, in den Kanal einführt.

6. Verfahren nach dem Anspruch 4, **dadurch gekennzeichnet, dass** man zur Realisierung einer intra-uterinen Vorrichtung, die mehrere wirksame Substanzen freigibt, aus verschiedenen Fasern, die, je nach den gewünschten Merkmale, jeweils eine der Substanzen freigibt, Faserstücke (51', 51'') ausschneidet, sie danach durch Kleben, in der gewünschten Reihenfolge auf Stoß miteinander verbindet, um den Körper (51) der intra-uterinen Vorrichtung zu bilden.

7. Zubehör zum Einsetzen einer intra-uterinen Vorrichtung nach irgendeinem der Ansprüche 1 bis 3, **dadurch gekennzeichnet, dass** er durch ein steril verpacktes Einführgerät (50) gebildet ist, der aus einem einen Innendurchmesser, der gleich oder etwas größer als der Außendurchmesser des besagten Körpers in T-Form ist, aufweisenden Rohr besteht, in dem der Körper der intra-uterinen Vorrichtung in T-Form untergebracht ist.
